# EUROPEAN PATENT APPLICATION

(11) **EP 4 574 077 A1**
(43) Date of publication of application: **25.06.2025**
(21) Application number: 23307305.5
(22) Date of filing: 21.12.2023
(51) Int. Cl.: A61B 34/20

(54) **METHOD AND SYSTEM FOR DETERMINING A CONFIGURATION OF A ROBOTIC ARM TO OPTIMIZE THE VISIBILITY OF A TRACKER**

(71) Applicant: Ecential Robotics, 38610 Gieres (FR)
(72) Inventor: SIEFFERT, Jérôme, 38610 GIERES (FR); RAHAL, Rahaf, 38610 GIERES (FR)
(74) Representative: Regimbeau

(57) **Abstract**

The invention relates to a method for determining a configuration of a robotic arm to guide a surgical tool according to at least one planned target while taking into account planning information and optimizing a visibility of a robot tracker by a localization system, the robot tracker being coupled to the robotic arm or to an end-effector, the end-effector being mechanically coupled to a distal end of the robotic arm and comprising a tool holder to accommodate the surgical tool, and a patient tracker being located in proximity to the region of interest, said method comprising: defining a patient's main axis; determining a relative position of the localization system with respect to the patient's main axis; for each of the at least one planned target: determine a relative position of a surgeon with respect to the patient's main axis using the planning information; determine an ideal configuration of the robotic arm using the determined relative position of the surgeon and of the localization system with respect to the patient's main axis. The invention also relates to a system for positioning the robotic arm in said determined configuration.

## Description

### FIELD OF THE INVENTION

The invention relates to a method for determining a configuration of a robotic arm to guide a surgical tool according to at least one planned target, with respect to a region of interest of a patient's body, while taking into account planning information and optimizing a visibility of a robot tracker by a localization system, the robot tracker being coupled to the robotic arm and a patient tracker being located in proximity to the region of interest. The invention also relates to a system for positioning the robotic arm in said determined configuration.

### BACKGROUND OF THE INVENTION

There is an increasing use of surgical robotic systems for assisting a user (e.g. a surgeon) during a surgical intervention. The surgical robotic system may guide the positioning and orientation of a surgical tool for the surgeon.

The surgical robotic system comprises a robotic arm having an end-effector with a tool holder to accommodate a surgical tool. The tool holder is to be placed with a given position and orientation relative to a planned target. The surgeon can couple a surgical tool, directly or through an access tube, to the tool holder so that once the tool holder is placed relative to the surgical target, the surgical robotic system assists the surgeon in the guiding of the tool. Thus, the surgeon may operate the surgical tool with the right position and orientation relative to the surgical target during the surgery on an anatomical structure of the patient, such as a bone.

For example, in spine surgery, the user may have to implant one or several screws into at least one vertebra. A robotic arm may assist the user by placing and maintaining the access tube of the tool holder according to a planned axis. The user may thus use a handheld drill passing through the access tube of the tool holder to drill a hole intended to receive the screw in a vertebra along the planned axis.

In this regard, a localization system that can localize trackers in real-time (high frequency, low latency) is used to carry out such manipulation, either in assistance of the surgeon or autonomously. Both the anatomical structure and the surgical tool can be localized, which allows determining the relative position of the surgical tool relative to the anatomical structure to be treated.

To that end, both the robotic arm and the anatomical structure may comprise a tracker rigidly attached thereto, each tracker being localizable by the localization system. The tracker attached to the robotic arm also allows indirect localization of the tool based on knowledge at any time of the kinematic model of the surgical robotic system between the tracker and the tool.

The tracker attached to the robotic arm may be coupled to the tool holder, or to the access tube of the tool holder, as disclosed in EP3821843. Alternatively, the markers of the tracker may be integrated to the end-effector and distributed around a central axis of the end-effector. The latter solution is advantageous since it improves the manipulability and reachability of the surgical tool by the surgeon, the tracker having a reduced overall size, and improves the visibility of the tracker thanks to the distribution of the markers around the central axis of the end-effector.

Precise localization of the robotic arm and the corresponding end-effector is relevant to successful surgical interventions, so it is very important to make sure that the tracker attached to the robotic arm is visible by the localization system to be able to properly guide the surgical tool.

Many variables can impact tracker visibility such as the position of the localization system in the operating room and the preferred placement of the surgeon with respect to the patient to operate. Additionally, the tracker may be visible, but the robotic arm may have a configuration which could hinder the surgeon in performing the planned operation and the surgeon may have to change his preferred placement and/or move the robotic arm to be able to properly do the planned operation.

This becomes more complex when a plurality of surgical targets is planned. It must be avoided that, when moving to the next surgical target, it turns out that there is no configuration of the robotic arm allowing acceptable visibility of the tracker and/or proper condition to do the planned operation. Indeed, in such a case the operation may need to be interrupted and resumed after adjusting the configuration of the operating room and/or the position of the surgeon. This prolongs the operating time and is therefore undesirable.

### BRIEF DESCRIPTION OF THE INVENTION

One aim of the invention is to propose a method for determining a configuration of the robotic arm to guide a surgical tool and a system for positioning the robotic arm in said configuration addressing part of those issues.

According to a first aspect, the invention is directed towards a method for determining a configuration of a robotic arm to guide a surgical tool according to at least one planned target with respect to a region of interest of a patient's body, using a localization system configured to localize (i) a robot tracker coupled to the robotic arm or to an end-effector mechanically coupled to a distal end of the robotic arm and comprising a tool holder to accommodate the surgical tool, and (ii) a patient tracker being located in proximity to the region of interest, said method comprising:
- defining a patient's main axis;
- determining a relative position of the localization system with respect to the patient's main axis;
- for each planned target:
   - determine a relative position of a surgeon with respect to the patient's main axis (M) using the planning information;
   - determine an ideal configuration of the robotic arm based on the determined relative position of the surgeon and of the localization system with respect to the patient's main axis to optimize a visibility of the robot tracker by the localization system.

By "optimizing the visibility" is meant in the present text providing the greatest possible visibility of the robot tracker, in particular by allowing the greatest possible number of markers to be visible by the localization system. By "ideal configuration" is meant the configuration of the robotic arm that provides said optimized visibility during surgery taking into account the position of the surgeon and of the localization system with respect to the patient and also the planning information. Otherwise said, the relative position of the surgeon and of the localization system, the planning information and the visibility of the robot tracker are technical constraints that have to be taken into account to determine the ideal configuration of the robotic arm.

In the present text, an "optimal configuration" is a configuration of the robotic arm different from the ideal configuration, which is determined if the ideal configuration is not valid, i.e. does not allow proper operation of the robotic arm. The optimal configuration provides a sufficient but poorer visibility of the robot tracker than the ideal configuration but is valid, i.e. allows operation of the robotic arm during surgery. Said optimal configuration is determined based on the ideal configuration in order to minimize the required adjustments.

Some preferred but non-limiting features of the method described above are the following, taken alone or in any technically feasible combination:
- defining the patient's main axis comprises defining an orientation of the patient's main axis and defining a relationship between the patient's main axis and the patient tracker;
- the planning information is used to define the patient's main axis;
- the method further comprises:
   ∘ generating or receiving a 3D volume of the region of interest;
   ∘ extracting anatomic information from the 3D volume and/or use an orbital axis of an imaging system, used to generate the 3D volume, to define the patient's main axis;
- determining the relative position of the localization system with respect to the patient's main axis comprises:
   ∘ determining a position of the localization system with respect to the patient tracker using the patient tracker, the patient tracker being localizable by the localization system;
   ∘ using the position of the localization system to determine whether the localization system is positioned on a head side or a foot side of the patient's main axis with respect to the patient tracker;
- the planning information comprises: a configuration of the patient's body on an operating table and, for each of at the least one planned target, a desired relative position of the surgeon with respect to the planned target; the method combines the planning information with a knowledge of the relative position of the planned target with respect to the patient's main axis to define the relative position of the surgeon with respect to the patient's main axis;
- determining the ideal configuration of the robotic arm comprises, for each planned target:
   ∘ determining a transitory configuration of the robotic arm wherein:
      ▪ a main tool holder axis, of the tool holder, is aligned with an axis of the planned target;
      ▪ a central axis of the end-effector is comprised in a plane formed by an axis of the planned target and an axis connecting a point of origin of the localization system with a patient's point of origin, the patient's point of origin being linked to the patient tracker;
   ∘ adjusting the transitory configuration, by rotating the robotic arm, in the transitory configuration, around the main tool holder axis by a predetermined angle, the direction of the rotation being defined using the relative position of the surgeon and of the localization system with respect to the patient's main axis;
- the method comprises, for each of the at least one planned target:
   ∘ verifying that the ideal configuration is a valid configuration of the robotic arm;
   ∘ otherwise, applying an adjustment step to determine an optimal configuration wherein the ideal configuration of the robotic arm is adjusted by rotating the robotic arm around the main tool holder axis by an additional rotation angle until a valid configuration is found, or a predefined maximal absolute value of the additional rotation angle is reached, if a valid configuration is found, the optimal configuration is defined as the valid configuration;
   - if the adjustment step does not provide a valid configuration, inviting a user to move the localization system and/or a base of the robotic arm and reapplying the method described above until a valid configuration is found;
- if the adjustment step does not provide a valid configuration, the method comprises, before inviting a user to move the localization system and/or the base of the robotic arm, an additional adjustment step, the additional adjustment step comprising modifying the desired relative position of the surgeon and reapplying the method described above;
- the method further comprises displaying on a user interface, for each planned target, a status of the configuration of the robotic arm for a current position of the localization system and of a base of the robotic arm, the status being chosen among:
   ∘ no issue;
   ∘ minor issue, wherein a valid configuration is found but requires modifying the desired relative position of the surgeon;
   ∘ major issue, wherein no valid configuration is found for the current position of the localization system and of the base of the robotic arm;
- the predetermined angle depends on the geometry of the robot tracker.

According to a second aspect, the invention is directed towards a system for positioning a robotic arm in a configuration to guide a surgical tool according to at least one planned target, with respect to a region of interest of a patient's body, said system comprising:
- a patient tracker rigidly attached in proximity to the region of interest;
- a robot tracker coupled to the robotic arm or to an end-effector;
- the localization system configured to localize the robot tracker and the patient tracker;
- a surgical robotic system comprising the robotic arm and an end-effector mechanically coupled to a distal end of the robotic arm, the end-effector comprising a tool holder to accommodate the surgical tool;
- a control unit coupled to the surgical robotic system and the localization system, the control unit storing planning information;
the system being characterized in that the control unit is configured to:
- implement the method described above to define an ideal or an optimal configuration of the robotic arm for each planned target;
- control at least one motor of the robotic arm to position the robotic arm in the associated ideal or optimal configuration for each planned target.

Some preferred but non-limiting features of the system described above are the following, taken alone or in any technically feasible combination:
- the robot tracker comprises a plurality of optical markers disposed on the end-effector and distributed around a central axis of the end-effector;
- the system further comprises an imaging system coupled to the control unit;
- the system further comprises a user interface coupled to the control unit, the control unit being configured to display on the user interface, for each planned target, a status of the configuration of the robotic arm for a current position of the localization system and of a base of the surgical robotic system.

### BRIEF DESCRIPTION OF THE DRAWINGS

Further features and advantages of the invention will be apparent from the following description, based on the appended drawings wherein:
- FIG. 1 is a general overview of a system according to the invention;
- FIG. 2 illustrates a surgical robotic system according to the invention and a patient tracker;
- FIG. 3 is a perspective view illustrating an end-effector according to the invention;
- FIG. 4 is a flowchart illustrating the method implemented by the control unit of the surgical robotic system for determining an ideal configuration of the robotic arm to guide a surgical tool according to at least one planned target, with respect to a region of interest of a patient's body, while taking into account planning information and optimizing a visibility of a robot tracker by a localization system;

For sake of legibility of the drawings, the figures are not necessarily drawn to scale.

The reference signs identical from one figure to another one designate the same elements or elements fulfilling the same functions.

### DETAILED DESCRIPTION OF EMBODIMENTS

The system 100 of the invention comprises a surgical robotic system 1, which can hold a surgical tool, and a localization system 3. The system 100 may also comprise an X-ray imaging system 2 and a user interface 40 such as presented below.

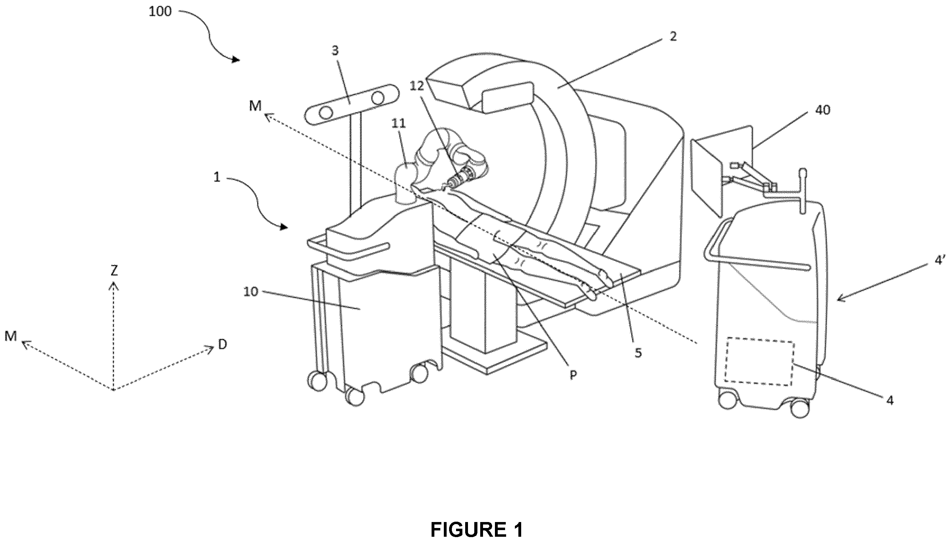

FIG.1 is a general overview of a system 100, according to the invention, to carry out a surgical intervention to treat an anatomical structure of a patient P lying on an operating table 5.

The surgical robotic system 1 comprises a base 10 and a robotic arm 11 carrying a surgical tool and is placed in the vicinity of the operating table 5.

The invention may comprise a user interface 40, which may include one or more screens, and an X-ray imaging system 2, both placed in the vicinity of the operating table 5. The user interface 40 is a link between the user and the system 100 and can be used for various purposes such as inputting and/or updating planning information, observing 2D images and 3D volumes obtained by the X-ray imaging system 2. The X-ray imaging system 2 is used to acquired 2D X-ray images of the anatomical structure to be treated. This X-ray imaging system 2 is not required to operate the system according to the invention.

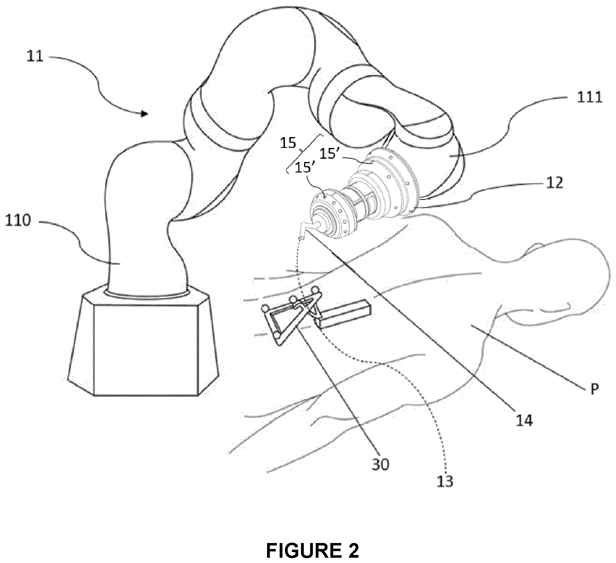

As shown in FIG.2, the robotic arm 11 comprises a tracker 15, and an additional tracker 30 may be rigidly attached to the patient P, for example via a mechanical or magnetic attachment, said trackers 15,30 being localized by the localization system 3.

A control unit 4 is coupled to the surgical robotic system 1, the localization system 3 and to the X-ray imaging system 2 and user interface 40 is available. The control unit 4 comprises at least one processor, configured to carry out the method of the invention that will be described in the following description, and at least one data storage unit to store the planning information.

### X-ray imaging system

The X-ray imaging system 2 comprises at least one X-ray source and at least one X-ray image detector. The X-ray imaging system 2 produces at least one 2D X-ray image that is the result of a conical projection of a patient anatomy, wherein the tip of the cone is approximately the central point of the X-ray source, and the basis of the cone is approximately the portion of the X-ray image detector that is reached by X-ray beams that have been collimated in a given shape and orientation.

For example, the X-ray imaging system 2 can be a conventional C-arm, or any Cone-Beam Computed Tomography (CBCT), such as the Surgivisio device (Surgivisio, Gieres, France), or Vision FD Vario 3D (Ziehm), CIOS Spin Mobile 3D (Siemens), Airo (Stryker), Loop-X (Brainlab), O-arm (Medtronic).

A conventional C-arm is designed to allow the X-ray source and X-ray detector to rotate along a C-shaped gantry while obtaining projection images of the patient P placed between the X-ray source and the X-ray detector of the gantry.

A CBCT has a mobile X-ray source and a mobile X-ray image detector, wherein the X-ray source and the X-ray image detector have motorized motions, moving together or independently. A CBCT can have a C-arm shape or an O-arm shape. It can be used to acquire a set of 2D X-ray images over approximately 180° of orbital rotation that can be combined with translations and from which a 3D volume can be reconstructed using tomography algorithms or tomosynthesis algorithms.

The X-ray imaging system 2 may be motorized, notably the C-shaped arm may comprise motors allowing movement horizontally, vertically and around the swivel axes, so that 2D X-ray images of the patient P are produced from almost any angle. Each motor is associated to an encoder that provides at any time the relative position of the X-ray imaging system 2 with respect to a reference position. When a 2D X-ray image is acquired, the corresponding position of the imaging system 2 is recorded. Thus, each 2D image is recorded in the referential of the imaging system 2.

In some embodiments, a 3D image of the patient P may be obtained during the surgery, using the X-ray imaging system 2 itself if it is a CBCT. Said 3D image is registered with respect to a tracker attached to the anatomical structure using known methods of calibration and navigation.

In other embodiments, the 3D image may be acquired prior to surgery using a Computed Tomography (CT) device or another CBCT device. Said 3D image of the patient is registered with respect to the tracker attached to the anatomical structure using a 3D registration method that can use many techniques, such as (i) a collection of surface points using the localizing system and their fitting on the anatomical structure like in the technique provided by 7D Surgical (North York, Canada), or (ii) the acquisition of 2D X-ray images calibrated with respect to the tracker attached to the anatomical structure and used for registration with the 3D image like in the technique provided in the MazorX robotic system (Medtronic), or (iii) any registration technique using localized ultrasound images, fiducials, anatomical points and the like.

### Surgical robotic system

A surgical robotic system 1, in the sense of the present invention, may comprise, with reference to FIG.1 and FIG.2:
- a base 10, which may be a movable cart (as shown in FIG.1) or may be attached to the operating table 5 (this embodiment has not been illustrated);
- a robotic arm 11 comprising a plurality of segments driven by motors, the robotic arm 11 having a proximal end 110 extending from the base, and a distal end 111 opposite to the proximal end;
- an end-effector 12 mechanically coupled to the distal end 111 of the robotic arm 11 and comprising a tool holder 14 to accommodate a surgical tool;
- a tracker called "robot tracker 15", coupled to the end-effector 14.

The robotic arm 11 comprises a plurality of degrees of freedom in translation and/or rotation and can be positioned according to a great variety of pose. Usually, the robotic arm 11 comprises at least five, and preferably six or seven motorized degrees of freedom. To that end, the robotic arm 11 comprises a plurality of articulated segments driven by motors. A robotic arm 11 can be for example the LBT Med^{™} robot provided by KUKA (Germany). Said robotic arm 11 can be controlled in an autonomous mode according to desired targets and trajectories, or it can be manipulated using a collaborative mode (cobot), or it can be telemanipulated using a master device, and a combination of these different modes can be used on the same surgical robotic system.

The surgical robotic system 1 can be active, in the sense that it holds and moves a powered surgical tool that interacts directly with the anatomical structure, or passive in the sense that it holds a guide in a predefined position with respect to the anatomical structure into which a surgical tool is inserted by a user. For example, a powered drill is mounted on the robotic arm tool holder 14 and actively drills the bone along a predefined path until an end point of a selected linear trajectory is reached. Or a screwdriver can be inserted in the tool holder 14 and manually be rotated by the surgeon to screw a screw in a previously drilled hole in the bone along a predefined path until an end point of a selected linear trajectory is reached.

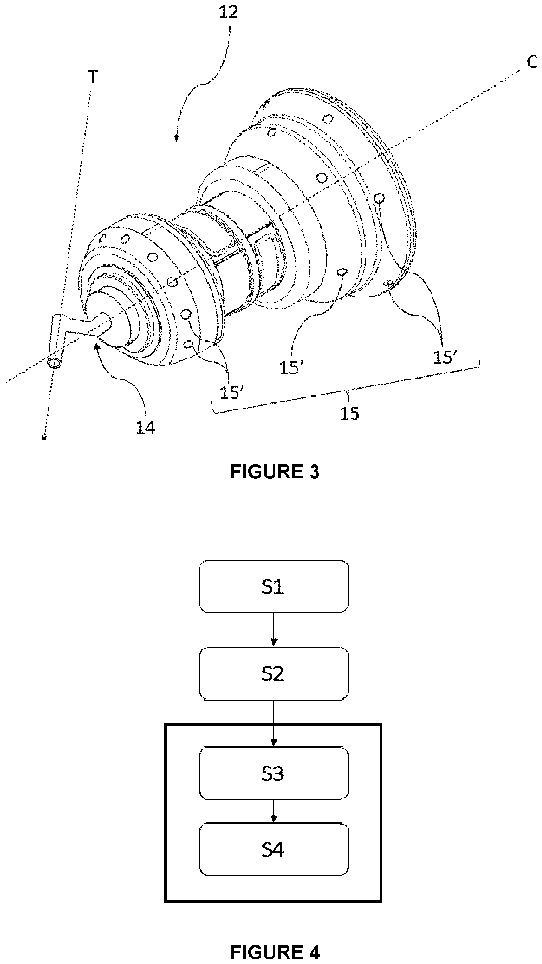

The tool holder 14, as illustrated in the FIG. 2 and FIG. 3, comprises an access tube to accommodate the surgical tool but other shape could be used, for example in the case of a powered tool, the tool holder could be a simple interface to fix the tool to the end-effector. The tool holder 14 comprises a main tool holder axis T which needs to be aligned with the axis of the planned to target to properly position the robotic arm 11 to maintain a tool according to the planned target. The main tool holder axis T corresponds to the axis of the tool once the tool is assembled to the tool holder 14. The main tool holder axis T is oriented toward the region of interest of the patient's body P when in use. In the example illustrated in FIG.2 and FIG.3, the tool holder 14 comprises an access tube and the main tool holder axis T corresponds to the longitudinal axis of the access tube.

The surgical tool is not illustrated in FIG. 2 but its position is represented schematically by reference 13.

Said surgical tool can be a powered drill, a powered burr or mill, an ultrasonic milling device, a radiofrequency or microwave or cryogenics ablation needle or a screwdriver, or any device that can interact with the anatomical structure to be treated. The surgical tool can move relative to the tool holder 14 in at least one degree of freedom, for example in two degrees of freedom: in translation along the tool axis and in rotation around the tool axis.

In a first embodiment, as illustrated in FIG.3, the robot tracker 15 comprises a plurality of optical markers 15' distributed around a central axis C of the end-effector 12.

In a second embodiment (not illustrated), the robot tracker 15 comprises a plurality of optical markers 15' distributed around a central axis of a segment of the robotic arm 11, the central axis of this segment being colinear with the central axis C of the end-effector 12. This segment of the robotic arm 11 is generally the distal end 111 of the robotic arm 11.

The optical markers 15' form groups of three or more optical markers 15' distributed around the central axis C of the end-effector 12. Each group of optical markers 15' enables localization of the robotic arm 11 by the localization system 3, independently of each other, preferably irrespective of the position and orientation of the robotic arm 11. Each group of optical markers 15' may have different configurations from each other, in terms of the number of optical markers 15' and/or arrangement of the optical markers 15' with respect to the end-effector 12. Advantageously more than one group of optical markers 15' is provided to provide a redundant design. Since each group of optical markers 15' enables precise localization of the robotic arm 11 independently, the tracking of the robotic arm 11 is not interrupted even if an object such as body fluid blocks a view of the sensor toward one of the optical markers.

The optical markers 15' can be active, such as light emitting elements or infrared transmitters, or passive, such as refractive objects having a substantially spherical or spheroidal shape.

In a one embodiment, illustrated in FIG.3, each optical marker 15' is provided in a cavity formed on the surface of the end-effector 12 in such a way that the optical marker 15' is substantially flush with the surface of the end-effector 12.

In another embodiment (not illustrated), some or all the optical markers 15', are disposed on a frame of one or more tracking arrays, the tracking arrays being removably attached to the end-effector 12.

Obviously, other configurations of the robot tracker 15 can be used such as combination of the two previously described embodiments.

### Control unit and stored planning data

In the embodiment of FIG.1, the control unit 4 is embedded in a separate station 4' from the surgical robotic system 1 and is configured to communicate wirelessly or by wires with the different elements of the system 100. This separate station 4' here also comprises the user interface 40 and may include batteries.

In other embodiments (not illustrated), the control unit may be embedded in the base 10 or the imaging system 2 if available.

The control unit 4 may be a single unit communicating with the different elements of the system 100 or correspond to a set of units communicating with each other, each unit controlling a respective element of the system 100. In the following description, the term "control unit" is used indifferently to designate one of these cases.

The control unit 4 comprises at least one processor configured to implement algorithms designed to carry out the method that will be described below. The control unit 4 also comprises at least one data storage device to store planning information and predetermined value of parameters of the method.

The control unit 4 is configured to:
- define a patient's main axis M;
- determine a relative position of the localization system 3 with respect to the patient's main axis M;
- for each planned target:
   ∘ determine a relative position of a surgeon with respect to the patient's main axis M using the planning information;
   ∘ determine a configuration of the robotic arm 11 using the determined relative position of the surgeon and of the localization system 3 with respect to the patient's main axis M;
   ∘ position the robotic arm 11 in said determined configuration.

The planning information may comprise:
- the configuration of the patient's body P on the operating table 5, such as whether the patient is in prone or supine position, or in a lateral right or lateral left position when the patient is in lateral decubitus position;
- the nature of the operation and/or of the region of interest, for example if it is a spine operation or a total knee replacement operation;
- at least one planned target, the planned target generally corresponding to the axis of insertion of a tool with respect to the region of interest,
- for each planned target, a desired relative position of the surgeon with respect to the planned target;
- the surgeon's mode of use of the robotic arm 11, i.e., whether the robotic arm 11 is used in right-handed mode or left-handed mode.

### Localization system

The localization system 3 is configured to localize the different trackers 15,30 used in the surgical site. The localization system 3 may be chosen among various technologies, such as optical localization (for example Aurora from NDI, Canada), electromagnetic localization, or ultrasound localization depending on the nature of the trackers used.

In the embodiment illustrated in FIG.1, the localization system 3 comprises a stereocamera arranged to detect optical markers 15'. As mentioned before, the optical markers 15' may be light emitting elements or passive optical markers. In FIG. 2, two trackers have been represented, the robot tracker 15 coupled to the end-effector 12, as previously described, and one tracker 30 rigidly attached, in proximity to the region of interest, to the patient P (also called "patient" tracker"). Optionally, more than one tracker 30 can be attached to the patient P to track different individual bones such as during a total knee replacement operation.

### Method for determining an ideal configuration of the robotic arm

FIG. 4 is a flowchart illustrating the method implemented by the control unit 4 of the system 100 to determine an ideal configuration of the robotic arm 11 to guide a surgical tool 14 according to at least one planned target, with respect to a region of interest of a patient's body, while taking into account planning information and optimizing a visibility of a robot tracker 15 by a localization system 3. In this flowchart, only the main steps have been represented. This flowchart is not intended to be limitative. In addition, steps that do not depend on each other may be interchanged, such as step S2 and step S3. Steps enclosed in the thick solid line frame are repeated for each planned target.

The method for determining the ideal configuration of the robotic arm 11 comprises the following steps:
- Step S1: defining a patient's main axis M;
- Step S2: determining a relative position of the localization system 3 with respect to the patient's main axis M;

For each planned target;
- Step S3: determining a relative position of a surgeon with respect to the patient's main axis M using the planning information;
- Step S4: determining the ideal configuration of the robotic arm 11 using the determined relative position of the surgeon and of the localization system 3 with respect to the patient's main axis M.

The patient's main axis M is an important axis used in step 2 and step 3. The patient's main axis M can be assimilated to the longitudinal axis of the patient's body P and may be linked to the anatomy which need to be operated, such as a spine axis or a leg axis. The patient's main axis M does not need to be precisely defined and is mostly used as reference to define the relative position of the various element involved in the method, in particular the position of the surgeon and the position of the localization system 3.

In step S1, the control unit 4 defines the patient's main axis M. This step also comprises defining the orientation of the patient's main axis M (from the foot to the skull of the patient) and defining the relationship between the patient's main axis M and a patient tracker 30 to be able to convert the information of the patient's main axis M in the chosen coordinate system to run the method and control the movement of the robotic arm 11. This chosen coordinate system is generally linked to a tracker and may be selected among the patient coordinate system (linked to the patient tracker 30), the robot coordinate system (linked to the robot tracker 15) or the coordinate system of the localization system 3 (which can localize each visible tracker 15,30). Obviously, it is possible to use different coordinate systems in the method and to convert one coordinate system to another using well-known methods.

In a first embodiment, the control unit 4 uses the stored planning information to define the patient's main axis M. For example, in the case of a spine surgery, the planned targets are generally the axis of the different screws that need to be implanted at an insertion point in a vertebra. If there are at least two vertebrae planned to be implanted, it is then possible to define the patient's main axis M as the axis passing through or closest to the insertion point of each screw. The more planned targets, the better defined the axis will be. Alternatively, instead of the insertion points, control points could be used. The control points are points manually placed during a preliminary step of the planning to identify the anatomy of the region of interest. In the case of a spine surgery, the control points allow to get access to the geometry of the spine. In addition, the vertebrae to be implanted are named following a convention, such as L1 to L5 for the five lumbar vertebrae, the smallest number corresponding to the vertebra closed to the skull. It is so possible to also define the patient's main axis orientation (generally from the foot to the skull) using the planning information. The relationship between the patient tracker 30 and the patient's main axis M is obtained using the planning information which contain the information of the position of the patient tracker 30 with respect to the region of interest and so to the planned targets.

In a second embodiment, the control unit 4 starts the acquisition, with the imaging system 2, of a plurality of 2D images of the region of interest of the patient's body P and generate a 3D volume of the region of interest using the plurality of 2D images. Alternatively, the 3D volume may have been preoperatively generated. In this case, the control unit 3 may receive the 3D volume from an external source. If the information about the orbital axis of the imaging system 2 used to acquire the 2D images is available, the patient's main axis M is defined as the orbital axis of the imaging system 2. If this information is not available, the control unit 4 is configured to extract anatomic information from the 3D volume, for example by using segmentation methods or other methods, to identify the visible bones, and their configuration, in the region of interest. The anatomic information can then be compared to an atlas to define the patient's main axis and the orientation of the patient's main axis with respect to the visible bones. In this embodiment, the patient P is generally equipped with a registration phantom (not visible in the figures), the registration phantom comprising radiopaque fiducials visible in the 3D volume and having a known relationship with the patient tracker 30. The information of the patient's main axis M is obtained in the coordinate system associated to the 3D volume and can then be converted into the patient coordinate system using the known relationship between the registration phantom and the patient tracker 30. The relationship between the patient tracker 30 and the patient's main axis M is thus obtained using the known relationship between the registration phantom and the patient tracker 30.

In a third embodiment, for example in the case of total knee replacement, the patient P is equipped with at least two patient trackers 30, one on each bone (tibia and femur), and the position of those patient trackers 30 is used to define the patient main axis M as the axis passing through, or closest to, the position of each patient trackers 30. The orientation of the patient's main axis M can be manually inputted by a user through the user interface 40, or defined automatically if the information on which tracker is attached to which bone is contained in the planning information. In this case, the position and orientation of the patient's main axis M is directly related to the patient's tracker 30 and so the relationship between the two is known.

In a fourth embodiment, if the position of the operating table 5 is known with respect to a tracker localizable by the localization system 2, the patient's main axis M is defined as the longitudinal axis of the operating table 5 on which the patient P rests. The orientation of the patient's main axis M can be manually input by a user through the user interface 40 or obtained using other means. In this embodiment, the operating table 5 may comprises its own tracker or has a known relationship with one element, equipped with a tracker, of the system 100. For example, the operating table 5 could be rigidly fixed to the base 10 of the surgical robotic system 1. In this case, the information of the patient's main axis M is obtained in the coordinate system of the tracker of the operating table 5, if available, or in that of the tracker which have a known relationship with the operating table 5. The relationship between the patient's main axis M and the patient tracker 30 is obtained by converting the information of the patient's main axis M into the coordinate system of the patient tracker 30 using the position information from the localization system 2 of the tracker, having a known relationship with the operating table 5, and the patient tracker 30.Obviously, others embodiment could be used to define to patient's main axis M.

In step S2, the control unit 4 determines a relative position of the localization system 3 with respect to the patient's main axis M. The localization system 3 provides the position of the patient tracker 30, in the coordinate system of the localization system 2, to the control unit 4. The control unit 4 then converts this information into the coordinate system of the patient tracker 30 using a rigid transformation to obtain the position of the localization system 3 with respect to the patient tracker 30. Once the position of the localization system 2, patient tracker 30, and patient main's axis M are expressed in the same coordinate system, it is then possible to determine whether the localization system is positioned on the upper half of the patient (also called "head side") or on the side of the bottom half of the patient (also called "foot side").

Advantageously the patient tracker 30 may be used as the point separating, along the patient's main axis, the upper half from the bottom half of the patient's body. If the localization system 3 has a position, along the patient's main axis M, higher than the position of the patient tracker 30, the localization system 3 will be defined as positioned on the head side with respect to the patient's main axis M. Otherwise, the localization system 3 will be defined as positioned on the foot side with respect to the patient's main axis M.

Alternatively, this information could be manually input but this requires an additional action from the user and will need to be manually updated if the position of the localization system 2 is modified at any moment.

In step S3, the control unit 4 determines, for each planned target, the relative position of a surgeon with respect to the patient's main axis M using the planning information. More particularly, the step S3 comprises determining if the surgeon is positioned to the left or to the right of the patient's main axis M using a reference plane to separate the left from the right. The reference plane is formed by the patient's main axis M and the vertical axis Z of the world (oriented toward the ceiling of the operating room), which information is obtained, for example, from the robot coordinate system.

A coordinate system may be created (as illustrated in FIG. 1) using the patient tracker 30, as the origin, the patient's main axis M and the vertical axis Z. The third axis D of this coordinate system may be obtained doing a vector product between the two other axes. If a point has a positive value along the third axis D, it will be considered as positioned to the right of the patient's main axis M.

This definition of left/right is independent of the configuration of the patient's body P. In other words, if the patient main's axis M is assimilated to the longitudinal axis of the operating table 5, this allows to know to which side of the surgical table 5 corresponds the desired position of the surgeon with respect to the planned target.

The planning information, as described before, comprise: the configuration of the patient's body P on the operating table 5 and, for each planned target, a relative desired position of the surgeon with respect to the planned target. If the configuration of the patient's body P is in lateral decubitus position, the surgeon generally stays on the same side of the patient for each target imposed by the type of operation, such as always on the back side of the patient P for a spine surgery.

Since in the planning information the preferred position of the surgeon is defined with respect to each target, and so depending on the configuration of the patient's body P, the control unit 4 needs to convert this information with respect to the patient's main axis M as previously explained. To do this, the control unit 4 combines the planning information with the knowledge of the relative position of the planned target with respect to the patient's main axis M. Indeed, the position of the planned targets are known with respect to the patient tracker 30 and the relationship between the patient tracker 30 and the patient's main axis M is known from step S1. The conversion information is summarized in the following table:

| Configuration of the patient's body | Preferred position of the surgeon with respect to the patient's body | Preferred position of the surgeon converted with respect to the patient's main axis M |
|---|---|---|
| Prone position | Left | Left |
| | Right | Right |
| Supine position | Left | Right |
| | Right | Left |
| Lateral right position | Back | Right |
| | Belly | Left |
| Lateral left position | Back | Left |
| | Belly | Right |

In step S4, the control unit 4 determines, for each planned target, the ideal configuration of the robotic arm 11 using the determined relative position of the surgeon and of the localization system 3 with respect to the patient's main axis M.

First, the control unit 4 determines a transitory configuration of the robotic arm 11 without considering the visibility of the robot tracker 15 by the localization system 3. In this transitory configuration, the main tool holder axis T is aligned with the axis of the planned target and the central axis C of the end-effector 12 is comprised in a plane formed by the axis of the planned target and an axis connecting a point of origin of the localization system 3 with a patient's point of origin, such as the distal end of the robotic arm 11 is oriented toward the localization system 3.

The patient's point of origin is linked to the patient tracker 30 and the point of origin of the localization system 3 may correspond to the barycenter of the different sensors of the localization system 3 or be arbitrary defined. For example, in the case of a stereo camera, the point of origin of the localization system 3 may correspond to the point in the middle of the two cameras.

In a first embodiment, the patient's point of origin corresponds to a particular point of the registration phantom. The position of the patient origin point is obtained through the patient tracker 30 and the known relationship between the patient tracker 30 and the registration phantom.

In a second embodiment, the patient's point of origin corresponds to a particular point of the patient tracker 30.

In a third embodiment, the patient's point of origin corresponds to a particular point of the region of interest of the patient's body, the patient tracker 30 having a known relationship with the region of interest. For example, in the case of spine surgery, the patient's point of origin can be a particular point of the vertebra onto which is fixed the patient tracker 30 or to another vertebra which has a known relationship with the vertebra onto which is fixed the patient tracker 30.

Once the transitory configuration is determined, the control unit 4 adjusts the transitory configuration by rotating the robotic arm 11 around the main tool holder axis T by a predetermined angle to obtain the ideal configuration of the robotic arm 11.

The absolute value of the predetermined angle is generally comprised between 45° and 135° and mostly depends on the geometry of the robot tracker 15. Ideally, the predetermined angle is determined such as at least one of the markers 15', of the robot tracker 15, is close to a 90° incidence with the localization system 3. This value may be determined arbitrary only considering the inclination of the markers 15' with respect to the central axis C, so independently of the position of the localization system 3. Alternatively, the precise position of the localization system 3, in particular its height position, could also be considered to obtain a more precise angle value but this required an additional step of computation and possible some delays since this angle value would need to be computed at the beginning of each operation and each time the height position of the localization system 3 is modified.

In a first example, the end-effector 12 comprises a cylindrical section on whose surface the markers 15' of the robot tracker 15 are disposed. In other words, the markers 15' are disposed on a surface having an inclination of 0° with respect to the central axis C of the end-effector 12. In this example the absolute value of the predetermined angle is preferentially equal to 90°.

In another example, illustrated in FIG.3, the end-effector 12 comprises several sections of truncated cones. The markers 15' of the robot tracker 15 are disposed on the inclined surface of the truncated cones, the inclined surfaces having an inclination of 30° with respect to the central axis C of the end-effector 12. In this example the absolute value of the predetermined angle is preferentially equal to 60°.

The direction of the rotation, clockwise (i.e. positive angle) or counterclockwise (i.e. negative angle), is defined using the relative position of the surgeon and of the localization system 3 with respect to the patient's main axis M. As a reminder, the main tool holder axis T is oriented toward the region of interest of the patient's body P when in use. The following table summarizes the choice of the direction of the rotation depending on the relative position of the surgeon and of the localization system 3 with respect to the patient's main axis M.

| Relative position of the surgeon | Relative position of the localization system | Direction of the rotation |
|---|---|---|
| Right | Head side | clockwise |
| Left | Head side | counterclockwise |
| Left | Foot side | clockwise |
| Right | Foot side | counterclockwise |

In this ideal configuration, the visibility of the robot tracker 15, by the localization system 3, is the best considering the position of the localization system 3 and the desired position of the surgeon.

However, the ideal configuration may not always correspond to a valid configuration of the robotic arm 11. For example, the ideal configuration may not be reachable by the robotic arm 11 without moving the base 10 of the surgical robotic system 1, or the ideal configuration could lead to a collision of any part of the surgical robotic system 1 with any other element of the system 100 or cross a predefined forbidden area delimited to protect the safety of the patient.

In this case, the configuration needs to be adjusted to provide a valid configuration (also called "optimal configuration") in which the visibility of robot tracker 15 by the localization system 3 is degraded, compared to the ideal configuration, but sufficient to allow a proper localization of the robot tracker 15. The control unit 4 is configured to apply an adjustment step wherein the configuration is adjusted starting from the ideal configuration by rotating the robotic arm 11 around the main tool holder axis T by an additional rotation angle until a valid configuration is found, or a predefined maximal absolute value of the additional rotation angle is reached.

Preferentially, the absolute value of the additional rotation angle is progressively increased, while alternating the direction of the rotation between each value increase, until a valid configuration is found or until the predefined maximal absolute value is reached.

Advantageously, there are two different predefined maximal absolute values, one for each direction of rotation, the predefined maximal absolute value associated to the direction of rotation used to reach the intermediary configuration being higher than the predefined maximal absolute value associated to the other direction. Indeed, when the end-effector is rotated back toward the transitory configuration, there is a higher risk of losing the visibility of the robotic tracker when the surgical tool is inserted in the tool holder 14 and manipulated by the surgeon.

For the example illustrated in FIG. 3 the additional rotation angle is comprised between -30° and 60° if the direction of the rotation to reach the ideal configuration was clockwise, otherwise between -60° and 30°.

If the adjustment step does not provide a valid configuration, the user is warned that no valid configuration has been found for the current position of the base 10 of the robotic system 1 and of the localization system 2. The user is then invited to move the localization system 2 and/or the position of the base 10 of the robotic arm 11 and the method is repeated until a valid configuration of the robotic arm 11 is found for each of the planned targets.

Optionally, when the adjustment step does not provide a valid configuration, the method comprises, before inviting the user to move the localization system 3 and/or the base 10 of the robotic arm 11, an additional adjustment step.

In this additional adjustment step, the control unit 4 is configured to modify the desired relative position of the surgeon for this planned target and to reapply the described method. For example, if the desired position of the surgeon is left, with respect to the main's patient axis M, the modified position will be right. In other words, the method changes the direction of the rotation used to obtain the ideal configuration.

If a valid configuration is found with the additional adjustment step, the control unit 4 may send a warning to the user to inform him that for this planned target he will have to modify his desired relative position. If the user accepts the modification of his desired relative position, this valid configuration is used as the optimal configuration of the robotic arm 11. Otherwise, the user is warned than no valid configuration has been found and is invited to move the base 10 of the robotic system 1 and/or the localization system 2 and the method is repeated until a valid configuration of the robotic arm 11 is found for each of the planned target.

This additional adjustment step is particularly useful when the patient P lies in a prone or supine position, the surgeon being most susceptible to accept to change his preferred position in this situation. Indeed, when the patient P lies in a lateral position the surgeon may not be able to change his preferred position if he needs to always be on the back side of the patient, to operate the spine for example. In this case it may not be useful to add this additional step since the surgeon is likely to refuse to change his preferred position.

Advantageously, the control unit 4 displays on the user interface 40 the state of the configuration of the robotic arm 11 for each planned target. For example, a green color could be associated to a planned target when the configuration of the robotic arm 11 is valid without changing the preferred position of the surgeon, a yellow color when the configuration of the robotic arm 11 is only valid by changing the preferred position of the surgeon, and a red color when no valid configuration of the robotic arm 11 exists without moving the localization system 2 and/or the position of the base 10 of the robotic arm 11. The user can, in near-real time, adjust, if required, the position of the different elements of the system 100 until the user interface 40 shows an acceptable result for each planned target. Obviously other means can be used to communicate to the user the state of the configuration of the robotic arm 11 for each planned target and a combination of means could be used such as using an audio signal in addition to the visual signal. For example, a first audio signal could be sent to the user when there is at least one invalid configuration (red color), and a second audio signal could be sent to the user when there are only valid configurations but at least one configuration requiring changing the preferred position of the surgeon.

This method is very advantageous since it allows to properly prepare the operating room by modifying, before starting the operation, the planning information and/or the position of some elements of the system 100, until there is a valid configuration of the robotic arm 11 compatible with the planned operation for each of the planned target. With this method, the operation will not be interrupted between two planned targets because of an invalid configuration of the robotic arm 11 since any possible problem will have been detected and addressed before starting the operation.

### Operation of the surgical robotic system

During a surgical intervention, the patient P lies on the operating table 5. In the case of spine surgery, the patient P may lie face down on the operating table 5, such that the region of interest, which may comprise one or several vertebrae, is accessible to the surgeon. In another example, not illustrated here, the patient P may lie on his side, in lateral decubitus position, on the operating table 5 for operation such as oblique lateral interbody fusion to have a better access to the lumbar spine. In the case of a spine surgery, the planned targets may correspond to the axis of the screw which needs to be inserted into the spine.

At the beginning of surgery, the patient P is equipped, in proximity to the region of interest, with a patient tracker 30 localizable by the localization system 3 and may be equipped with a registration phantom (not visible in the figures) if a 3D volume acquisition of the region of interest is needed. The patient tracker 30 has a known relationship with the registration phantom.

If the robot tracker 15 is not incorporated to the end-effector 12 of the robotic arm 11, the robot tracker 15 is mounted to the end-effector 12 of the robotic arm 11. The relationship between the robot tracker 15 and the surgical robotic system 1 is known, the robot tracker 15 having a known position on the surgical robotic system 1. This robot tracker 15 is also localizable by the localization system 3 and allow the control unit 4 to know the position of any part of the surgical robotic system 1 with respect to the patient tracker 30 and so the region of interest. This is possible using the information from the localization system 3, the relationship between the robot tracker 15 and the surgical system 1, and the encoder values of each motor of the robotic arm 11.

A 3D image may be acquired either at the beginning of the surgery using the X-ray imaging system itself (CBCT) or prior to the surgery with another imaging system (CT or CBCT).

The control unit 4 implements the previously described method to determine the ideal configuration of the robotic arm 11 to guide the surgical tool according to the at least one planned target, with respect to the region of interest of the patient's body P, while taking into account planning information and optimizing the visibility of the robot tracker 15 by the localization system 3. The control unit 4 displays on the user interface 40 the status of the configuration of the robotic arm 11 for each planned target using for example a color code. The color code for a target may be green for no issue, orange for minor issue (require changing the preferred position of the surgeon) and red for major issue (require moving the position of the localization system 3 and/or the base 10 of the surgical robotic system 1).

If there is no issue (green color) for any planned target, the surgeon can start the planned operation.

If there is at least one minor issue (orange color) but no major issue (red color), the surgeon needs to decide if he accepts to modify his preferred position for the problematic target. If the surgeon accepts to modify his preferred position for each planned target classified as minor issue, the surgeon can start the planned operation. If the surgeon cannot, or do not wish to modify his position, the surgeon (or another user) moves the base 10 of the surgical robot system 1 and/or the localization system 3 until there is no issue or only minor issue for targets for which the surgeon accepts to modify his preferred position before being able to start the planned operation.

If there is a major issue (red color) for at least one of the planned targets, the surgeon needs to move the base 10 of the surgical robot system 1 and/or the localization system 3 until there is no more major issue, and minor issue for which the surgeon cannot, or do not want to modify his preferred position, before being able to start the planned operation.

Once the surgeon starts the planned operation, the control unit 4 positions the robotic arm 11, by sending appropriate command to each of the motors of the robotic arm 11, in the ideal or optimal configuration for the first planed target and the surgical tool is inserted in the tool holder 12. Once the first target is resolved, the control unit 4 moves the robotic arm 11 in the ideal or optimal configuration of the next target and repeats this for each planned target.

In the above-described method, the planned targets are mostly described as the axis of insertion of the tool with respect to the region of interest. However, the planned targets may also include, for at least one planned target, a planned cutting plane. In this case, the tool used is generally a surgical saw whose blade must be aligned with the planned cutting plane. To use the described method in this case, the robotic arm may require a specific structure where the saw blade (including the motor and its handle) can move in rotation with the end-effector. In this case, the step S4 need to be adjusted to align the saw blade with the planned cutting plane, to obtain the transitory configuration, and to rotate the robotic arm around the rotation axis of the saw with the end-effector, instead of the tool holder axis, to adjust the transitory configuration while not moving the position and orientation of the saw blade.

In addition, in the examples here described, the patient's main axis M is oriented from the feet to the skull of the patient, but the opposite could also be selected with some obvious adjustments to the method. For example, in step 2 the localization system will be defined as positioned on the head side if the localization system has a position, along the patient's main axis, lower (instead of higher) than the position of the patient tracker.

## Claims

1. Method for determining a configuration of a robotic arm (11) to guide a surgical tool (14) according to at least one planned target with respect to a region of interest of a patient's body (P), using a localization system configured to localize (i) a robot tracker (15) coupled to the robotic arm (11) or to an end-effector (12) mechanically coupled to a distal end (111) of the robotic arm (11) and comprising a tool holder (14) to accommodate the surgical tool (14), and (ii) a patient tracker (30) located in proximity to the region of interest, said method comprising:
- defining a patient's main axis (M);
- determining a relative position of the localization system (3) with respect to the patient's main axis (M);
- for each planned target:
* determine a relative position of a surgeon with respect to the patient's main axis (M) using planning information;
* determine an ideal configuration of the robotic arm (11) based on the determined relative position of the surgeon and of the localization system (3) with respect to the patient's main axis (M) to optimize a visibility of the robot tracker (15) by the localization system (3).

2. Method according to claim 1, wherein defining the patient's main axis (M) comprises defining an orientation of the patient's main axis (M) and defining a relationship between the patient's main axis (M) and the patient tracker (30).

3. Method according to any of claims 1 to 2, wherein defining the patient's main axis (M) comprises using the planning information.

4. Method according to any of claims 1 to 2, further comprising:
- generating or receiving a 3D volume of the region of interest;
- extracting anatomic information from the 3D volume and/or use an orbital axis of an imaging system (2) used to generate the 3D volume, to define the patient's main axis (M).

5. Method according to any of claims 1 to 4, wherein determining the relative position of the localization system (3) with respect to the patient's main axis (M) comprises:
- determining a position of the localization system (3) with respect to the patient tracker (30) using the patient tracker (30), the patient tracker (30) being localizable by the localization system (3);
- using the position of the localization system (3) to determine whether the localization system (3) is positioned on a head side or a foot side of the patient's main axis (M) with respect to the patient tracker (30).

6. Method according to any of claims 1 to 5, wherein:
- the planning information comprises: a configuration of the patient's body (P) on an operating table (5) and, for each planned target, a desired relative position of the surgeon with respect to the planned target;
- determining the relative position of the surgeon with respect to the patient's main axis (M) comprises combining the planning information with a knowledge of the relative position of the planned target with respect to the patient's main axis (M).

7. Method according to any of claims 1 to 6, wherein determining the ideal configuration of the robotic arm (11) comprises, for each planned target:
- determining a transitory configuration of the robotic arm (11) wherein:
* a main tool holder axis (T) of the tool holder (14) is aligned with an axis of the planned target;
* a central axis (C) of the end-effector (12) is comprised in a plane formed by an axis of the planned target and an axis connecting a point of origin of the localization system (3) with a patient's point of origin, the patient's point of origin being linked to the patient tracker (30);
- adjusting the transitory configuration, by rotating the robotic arm (11), in the transitory configuration, around the main tool holder axis (T) by a predetermined angle, the direction of the rotation being defined using the relative position of the surgeon and of the localization system (3) with respect to the patient's main axis (M).

8. Method according to claim 7 wherein the method comprises, for each planned target:
- verifying that the ideal configuration is a valid configuration of the robotic arm (11);
- otherwise, applying an adjustment step to determine an optimal configuration wherein the ideal configuration of the robotic arm (11) is adjusted by rotating the robotic arm (11) around the main tool holder axis (T) by an additional rotation angle until a valid configuration is found, or a predefined maximal absolute value of the additional rotation angle is reached, if a valid configuration is found, the optimal configuration is defined as the valid configuration;
- if the adjustment step does not provide a valid configuration, inviting a user to move the localization system (3) and/or a base (10) of the robotic arm (11) and reapplying the method of any of claims 1 to 7 until a valid configuration is found.

9. Method for determining an optimal configuration of a robotic arm (11) according to claim 8, further comprising, if the adjustment step does not provide a valid configuration, an additional adjustment step before inviting a user to move the localization system (3) and/or the base (10) of the robotic arm (11), the additional adjustment step comprising modifying the desired relative position of the surgeon and reapplying the method of any of claims 1 to 8.

10. Method according to any of claims 8 to 9, further comprising displaying on a user interface (40), for each planned target, a status of the configuration of the robotic arm (11) for a current position of the localization system (3) and of a base (10) of the robotic arm (11), the status being chosen among:
- no issue;
- minor issue, wherein a valid configuration is found but requires modifying the desired relative position of the surgeon;
- major issue, wherein no valid configuration is found for the current position of the localization system (3) and of the base (10) of the robotic arm (11).

11. Method according to any of claims 7 to 10, wherein the predetermined angle depends on the geometry of the robot tracker (15).

12. System (100) for positioning a robotic arm (11) in a configuration to guide a surgical tool according to at least one planned target, with respect to a region of interest of a patient's body (P), said system comprising:
- a patient tracker (30) rigidly attached in proximity to the region of interest;
- a robot tracker (15) coupled to the robotic arm or to an end-effector;
- a localization system (3) configured to localize the robot tracker (15) and the patient tracker (30);
- a surgical robotic system (1) comprising the robotic arm (11) and an end-effector (12) mechanically coupled to a distal end (111) of the robotic arm (11), the end-effector comprising a tool holder (14) to accommodate the surgical tool;
- a control unit (4) coupled to the surgical robotic system (1) and the localization system (3), the control unit (4) storing planning information;
the system being **characterized in that** the control unit (4) is configured to :
- implement the method of any of claims 1 to 11 to define an ideal or an optimal configuration of the robotic arm for each planned target;
- control at least one motor of the robotic arm to position the robotic arm (11) in the associated ideal or optimal configuration for each planned target.

13. System (100) according to claim 12, wherein the robot tracker (15) comprises a plurality of optical markers (15') disposed on the end-effector (14) and distributed around a central axis (C) of the end-effector (14).

14. System (100) according to any of claims 12 to 13, further comprising an imaging system (2) coupled to the control unit (4).

15. System (100) according to any of claims 12 to 14, further comprising a user interface (40) coupled to the control unit (4), the control unit (4) being configured to display on the user interface (40), for each planned target, a status of the configuration of the robotic arm (11) for a current position of the localization system (3) and of a base (10) of the surgical robotic system (1).
